# EUROPEAN PATENT APPLICATION

(11) **EP 4 678 172 A1**
(43) Date of publication of application: **14.01.2026**
(21) Application number: 24766373.5
(22) Date of filing: 01.03.2024
(51) Int. Cl.: A61K 31/416, A61P 35/00, C07D 231/56

(54) **USE OF NAPHTHYLAMIDE COMPOUND IN TREATMENT OF MENINGIOMA**

(30) Priority: 03.03.2023 CN 202310198273
(71) Applicant: Shanghai Runshi Medical Technology Co., Ltd, Shanghai 200040 (CN); Shanghai Institute of Materia Medica, Chinese Academy of Sciences, Shanghai 201203 (CN)
(72) Inventor: LI, Wenbin, Shanghai 200040 (CN); KANG, Zhuang, Shanghai 200040 (CN); HUANG, Wei, Shanghai 200040 (CN); LI, Shenglan, Shanghai 200040 (CN); DING, Jian, Shanghai 201203 (CN); LIU, Guangping, Shanghai 200040 (CN); DUAN, Wenhu, Shanghai 201203 (CN); LIU, Chenxuan, Shanghai 200040 (CN); LAN, Yanjie, Shanghai 200040 (CN); CHEN, Xuetao, Shanghai 200040 (CN); XIE, Hua, Shanghai 201203 (CN); XIANG, Silong, Shanghai 200040 (CN); YANG, Xue, Shanghai 200040 (CN); SHE, Fenglin, Shanghai 200040 (CN); SU, Yangzhi, Shanghai 200040 (CN); YANG, Yang, Shanghai 200040 (CN)
(74) Representative: Gille Hrabal Partnerschaftsgesellschaft mbB Patentanwälte
(86) International application number: PCT/CN2024/079683
(87) International publication number: WO 2024/183657

(57) **Abstract**

The present invention provides a use of a naphthylamide compound (I) or a pharmaceutically acceptable salt thereof in preparation for a drug for treating meningioma, and provides a corresponding treatment method. The structure of the compound (I) is as shown below. The compound (I) has an excellent inhibitory effect on meningioma, can effectively treat meningioma, and has good safety and tolerance.

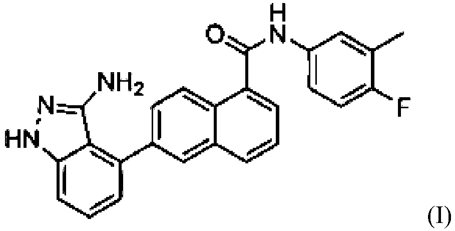

## Description

### TECHNICAL FIELD

The present application relates to the field of medicines, and particularly relates to use of a naphthylamide compound or a pharmaceutically acceptable salt thereof in the manufacture of a medicament for treating meningioma.

### BACKGROUND

Meningiomas (tumors of the meninges) are the most common primary intracranial tumors, accounting for about two-fifths of primary intracranial tumors. According to different sites of occurrence, meningioma is categorized into intracranial meningioma and ectopic meningioma, in which the intracranial meningioma is derived from intracranial arachnoid cells, and the ectopic meningioma refers to a meningioma occurring in tissues and organs not covered by meninges and is mainly derived from residual arachnoid tissues in the embryonic period. The annual morbidity of meningioma is approximately 7.6/100,000. Patients were relatively young at the time of initial diagnosis, with 77.7% of the patients aged 20-44 years.

According to growth sites, meningioma can be categorized into convexity meningioma, parafalcine meningioma, olfactory groove meningioma, tuberculum sellae meningioma, sphenoid ridge meningioma, parasagittal meningioma, middle cranial fossa meningioma, clival meningioma, foramen magnum meningioma, etc. According to clinical and pathological characteristics of meningioma, meningioma is categorized by the World Health Organization (WHO) into 3 grades: benign meningioma (WHO grade I), untypical meningioma (WHO grade II), and malignant meningioma (WHO grade III). Benign meningioma (WHO grade I) accounts for about 65%-80% of all meningiomas, and is categorized into the following 9 subtypes: 1: meningothelial meningioma, 2: fibrous (fibroblastic) meningioma, 3: transitional (mixed) meningioma, 4: psammomatous meningioma, 5: angiomatous meningioma, 6: microcystic meningioma, 7: secretory meningioma, 8: lymphoplasmacyte-rich meningioma, and 9: metaplastic meningioma. Most of these meningiomas exhibit slow growth and a low recurrence rate after surgery. Untypical meningioma (WHO grade II) accounts for about 20%-35% of all meningiomas, and is categorized into the following 3 subtypes: 1: atypical meningioma, 2: clear cell meningioma, and 3: chordoid meningioma. Malignant meningioma (WHO grade III) accounts for about 3% of all meningiomas, and is categorized into the following 3 subtypes: 1: rhabdoid meningioma, 2: papillary meningioma, and 3: anaplastic meningioma. The meningiomas grading WHO grades II-III are high-grade, and are characterized by high invasiveness, low differentiation, and high recurrence and metastasis.

Meningioma may be accompanied by neurofibromatosis and brings complications including visual, visual field, olfactory, or auditory disorders, as well as limb movement disorders, etc. Vision loss is associated with the primary site of the tumor. In most cases, symptoms of meningioma that occurs in the optic canal develop early and are severe. Papilledema occurs at the early stage and, after a long-term progression, develops into secondary optic atrophy and tubular meningioma. Meningioma results in visual field contraction at the early stage, and meningioma that occurs in the orbital apex may lead to scotomas within the visual field.

The overall ten-year survival rate for meningioma is 57.1%. Patients with WHO grade II meningioma have a 5-year recurrence rate of about 50%, and patients after recurrence have a 10-year survival rate of 53%; patients with WHO grade III meningioma have a 5-year recurrence rate of about 90%, and patients after recurrence have a 10-year survival rate of 0%.

Meningioma is usually diagnosed using brain MRI, and patients are stratified according to the presence or absence of symptoms and tumor size. Treatment recommendations are generally in accordance with the tumor grading.

Patients with asymptomatic small tumors (≤ 3 cm) may undergo regular follow-up. In other cases, patients should receive surgical resection as much as possible, and if they do not have surgical conditions, radiotherapy may be selected. Patients with grade I meningioma may only undergo regular follow-up (conservative treatment) after surgery, and postoperative radiotherapy is considered for patients with symptoms.

Postoperative radiotherapy is recommended for patients with grade II meningioma that cannot be completely resected. Certain patients (e.g., those not suitable for radiotherapy) may undergo regular follow-up. Postoperative radiotherapy may be considered for patients with grade II meningioma after complete surgical resection. All patients with grade III meningioma after surgical resection shall receive adjuvant radiotherapy to strengthen local control.

For meningioma that has recurred after surgery and/or radiotherapy or is not suitable for surgery or radiotherapy, systemic treatment is generally considered, but there are no recognized standard treatment means. This therapeutic field lacks large randomized trials. Typically, ORRs of systemic treatment for meningioma are very low. In a review of the Response Assessment in Neuro-Oncology (RANO) Working Group on the outcomes of drug therapy for meningioma, 37 out of 42 publications reported imaging efficacy data. Of the 555 patients involved in these 37 reports, the best imaging responses were reported as 1 CR (0.2%), 10 PR (1.8%), 13 MR (2.3%), and 343 SD (62%). The combined response rate of CR + PR + MR was 4.3%, indicating that tumor shrinkage was rarely observed. PFS-6 (6-month PFS rate) or ORR is often used as the clinical trial endpoint in this therapeutic field. The RANO working group assessed the historical data of clinical trials regarding drug therapy for meningioma. WHO grade I meningioma had a weighted PFS6 of 29% (95% CI: 20.3%-37.7%). WHO grade II/III meningioma had a weighted average PFS6 of 26% (95% confidence interval: 19.3%-32.7%). In addition, in several small clinical trials, over 20% and even 60% of the patients exhibited grade 3 or higher toxicity response, or stopped the treatments due to drug toxicity.

Therefore, a safe and effective drug for the treatment of meningioma is urgently needed to solve the problem of drug unavailability clinically at present.

Compound (I) was first disclosed in CN104860885A and has a structure as shown in the following formula (I),

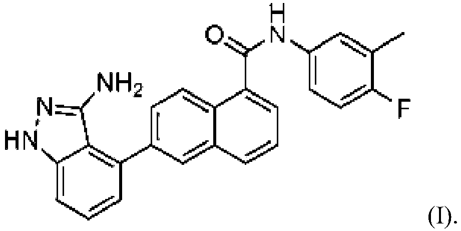

Compound (I) is a VEGFR/CSF1R dual-target inhibitor with excellent activity, whose therapeutic effect on meningioma, however, is not yet studied.

### SUMMARY

The inventors of the present application have unexpectedly found that compound (I) has excellent inhibitory activity against meningioma cells through long-term and intensive studies, and preliminary clinical studies have shown that compound (I) is capable of effectively treating meningioma and exhibits good safety and tolerance, and has the prospect of being developed as an anti-meningioma drug.

In a first aspect of the present application, provided is use of a compound (I) or a pharmaceutically acceptable salt thereof in the manufacture of a medicament for treating meningioma, wherein the compound (I) has the following structure:

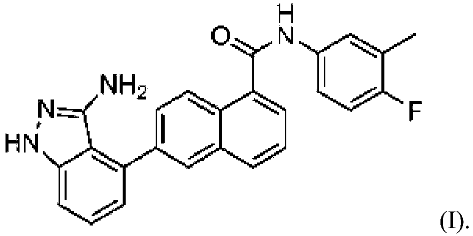

In a second aspect of the present application, provided is a pharmaceutical composition for treating meningioma, comprising a therapeutically effective amount of a compound (I) or a pharmaceutically acceptable salt thereof and a pharmaceutically acceptable carrier, wherein the compound (I) has the following structure:

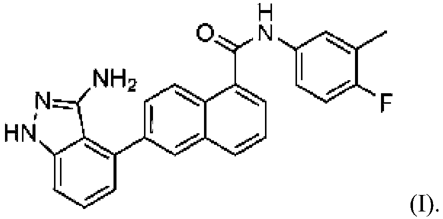

In a third aspect of the present application, provided is a method for treating meningioma in an individual, comprising:
administering to an individual having meningioma a therapeutically effective amount of a compound (I) or a pharmaceutically acceptable salt thereof or a pharmaceutical composition comprising the compound (I) or the pharmaceutically acceptable salt thereof, wherein the compound (I) has the following structure:

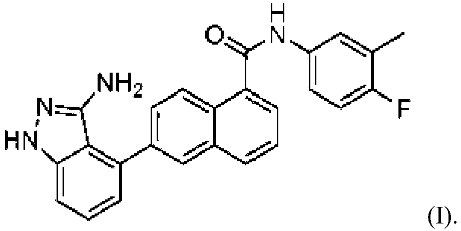

In a fourth aspect of the present application, provided is a compound (I) or a pharmaceutically acceptable salt thereof, or a pharmaceutical composition comprising the compound (I) or the pharmaceutically acceptable salt thereof for use in the treatment of meningioma, wherein the compound (I) has the following structure:

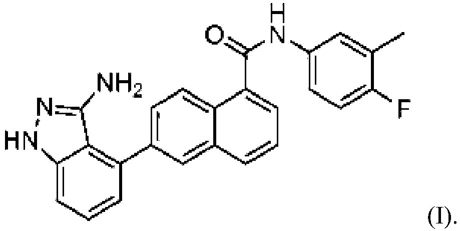

In some embodiments of the present application, the meningioma in the first to the fourth aspects described above includes, but is not limited to, WHO grade I meningioma, WHO grade II meningioma, and WHO grade III meningioma categorized according to clinical and pathological characteristics.

In some embodiments, the WHO grade I meningioma is a benign meningioma, including but not limited to: meningothelial meningioma, fibrous meningioma, transitional meningioma, psammomatous meningioma, angiomatous meningioma, microcystic meningioma, secretory meningioma, lymphoplasmacyte-rich meningioma, and metaplastic meningioma. In a specific embodiment, the meningioma is selected from one or more of meningothelial meningioma, fibrous meningioma, transitional meningioma, psammomatous meningioma, angiomatous meningioma, microcystic meningioma, secretory meningioma, lymphoplasmacyte-rich meningioma, and metaplastic meningioma.

In some embodiments, the WHO grade II meningioma is an untypical meningioma, including but not limited to: atypical meningioma, clear cell meningioma, and chordoid meningioma. In a specific embodiment, the meningioma is selected from one or more of atypical meningioma, clear cell meningioma, and chordoid meningioma.

In some embodiments, the WHO grade III meningioma is a malignant meningioma, including but not limited to: rhabdoid meningioma, papillary meningioma, and anaplastic meningioma. In a specific embodiment, the meningioma is selected from one or more of rhabdoid meningioma, papillary meningioma, and anaplastic meningioma.

In some embodiments of the present application, the meningioma in the first to the fourth aspects described above includes intracranial meningioma and ectopic meningioma categorized according to sites of occurrence.

In some embodiments of the present application, the meningioma in the first to the fourth aspects described above includes, but is not limited to, convexity meningioma, parafalcine meningioma, olfactory groove meningioma, tuberculum sellae meningioma, sphenoid ridge meningioma, parasagittal meningioma, middle cranial fossa meningioma, clival meningioma, and foramen magnum meningioma categorized according to growth sites.

In some embodiments of the present application, the meningioma in the first to the fourth aspects described above is a meningioma that failed treatment with other therapeutic means or recurred (also referred to as a recurrent or refractory meningioma).

In some embodiments, the other therapeutic means include, but are not limited to: surgery, radiotherapy, and treatment with an anti-tumor drug.

In some embodiments, the anti-tumor drug includes, but is not limited to: a chemotherapeutic drug, a targeted therapeutic drug, an immunotherapeutic drug, etc., such as camrelizumab, bevacizumab, sunitinib, everolimus, etoposide, temozolomide, sirolimus, honokiol, etc. In a specific embodiment, the anti-tumor drug is selected from one or more of camrelizumab, bevacizumab, sunitinib, everolimus, etoposide, temozolomide, sirolimus, and honokiol.

In some embodiments of the present application, the pharmaceutical composition in the second to the fourth aspects described above is formulated into a clinically acceptable pharmaceutical dosage form, such as an oral formulation, preferably an oral solution.

In some embodiments of the present application, the therapeutically effective amount in the third aspect described above is a daily administration dose of the compound (I) of 5 mg-30 mg, preferably 10 mg-25 mg, and more preferably 10 mg-20 mg or 15 mg-25 mg. In a specific embodiment, the daily administration dose of the compound (I) is 10 mg, 15 mg, 20 mg, or 25 mg.

In some embodiments of the present application, the compound (I) in the first to the fourth aspects described above is administered in a single dose once daily, in multiple doses daily, or at intervals. In a preferred embodiment, the compound (I) is administered in a single dose once daily. In some embodiments of the present application, the compound (I) in the first to the fourth aspects described above has an administration cycle of 21 consecutive days; optionally, the administration may be repeated for multiple cycles.

In some embodiments of the present application, the individual in the third aspect described above refers to a mammal, including but not limited to a primate, a cow, a horse, a pig, a sheep, a goat, a dog, a cat, as well as a rodent such as a rat and a mouse. In a preferred embodiment, the individual is a human.

It should be understood that within the scope of the present application, the technical features described above of the present application and the technical features specifically described hereinafter (e.g., in the examples) may be combined with each other to constitute new or preferred technical solutions. Due to limited space, such solutions are not described herein.

As used herein, the term "pharmaceutically acceptable salt" refers to a salt formed by the compound of the present application with an acid or a base that is suitable for use as a drug. Pharmaceutically acceptable salts include inorganic and organic salts.

"Pharmaceutically acceptable carrier" refers to a carrier that does not interfere with the biological activity of an active ingredient and includes those conventionally used in the pharmaceutical field, such as one or more compatible solid or liquid fillers or gel materials, which are suitable for use in a mammal, specifically a human, and must be of sufficient purity and sufficiently low toxicity. "Compatible" herein means that the components of the composition are capable of intermixing with the compound of the present application and with each other, without significantly diminishing the pharmaceutical efficacy of the compound.

The administration mode of the pharmaceutical composition of the present application is not particularly limited. In some embodiments, exemplary administration modes include, but are not limited to: oral administration, intratumoral administration, rectal administration, parenteral (intravenous, intramuscular, or subcutaneous) administration, and topical administration.

Correspondingly, the drug of the present application may be formulated into various clinically acceptable pharmaceutical dosage forms. In some embodiments, the dosage forms described above include an oral dosage form, an injectable dosage form, a topical dosage form, an external dosage form, etc., preferably an oral dosage form such as a solid dosage form or an oral liquid, and more preferably an oral solution.

The solid dosage form such as a tablet, a sugar pill, a capsule, a pill, and a granule may be manufactured using a coating and a shell such as an enteric coating and other materials well known in the art. They may comprise an opacifying agent, and the active compound in such a composition may be released in a certain part of the digestive tract in a delayed manner. If necessary, the active compound may also be formulated into a microcapsule form with one or more of the excipients described above.

The oral liquid dosage form may include a solution, a syrup, an emulsion, a suspension, etc., preferably an emulsion, and more preferably a self-emulsifying formulation. The auxiliary material for the self-emulsifying formulation comprises an oily phase, an emulsifier, and a co-emulsifier and may further comprise an antioxidant. The oily phase comprises a medium-chain triglyceride (MCT), glyceryl monocaprylate, glyceryl dicaprylate, glyceryl monooleate, glyceryl monolinoleate, corn oil, castor oil, sesame oil, peanut oil, olive oil, etc. The emulsifier comprises polyoxyethylene (40) hydrogenated castor oil (RH40), polyoxyethylene (35) castor oil (Cremophor EL), vitamin E polyethylene glycol succinate (TPGS 1000), polyethylene glycol (15)-hydroxystearate (HS15), linoleoyl polyoxyl-6 glyceride, caprylocaproyl polyoxyl glyceride (Labrasol ALF), etc. The co-emulsifier is selected from: polyethylene glycol (PEG), diethylene glycol monoethyl ether (Transcutol HP), propylene glycol, glycerin, etc. The antioxidant is selected from: dibutyl hydroxy toluene, butylhydroxyanisole, vitamin E, and propyl gallate. The self-emulsifying formulation may also be formulated into a capsule or a tablet. Examples of the oral solution are described in the detailed description of the present application.

The composition for parenteral injection may comprise a physiologically acceptable sterile aqueous or anhydrous solution, dispersion, suspension, or emulsion, and a sterile powder for reconstitution into a sterile injectable solution or dispersion.

The dosage form of the compound of the present application for topical administration comprises an ointment, a powder, a patch, an aerosol, and an inhalant. The active ingredient is mixed under sterile conditions with a physiologically acceptable carrier containing any preservative, buffering agent, or propellant that may be required if necessary.

The compound of the present application may be administered separately, or in combination with an additional pharmaceutically acceptable anti-tumor drug (e.g., chemotherapeutic drugs, targeted therapeutic drugs, and immunotherapeutic drugs), or an additional treatment means (e.g., radiotherapy and surgery).

The therapeutically effective amount of the present application refers to a pharmaceutically effective administration dose, namely an amount of the active compound sufficient to significantly improve the condition without causing serious side effects. A specific dose and administration frequency can be determined by a skilled physician according to conventional skills allowing for factors such as the route of administration, the health condition of the patient, etc. In the first dose escalation study (phase I trial) in a human, the most sensitive animal species were selected to calculate the maximum recommended starting dose for clinical trials according to the pre-clinical toxicology study. The starting dose was calculated according to the calculation methods recommended by "Technical Guidelines for Non-Clinical Research on Anti-Cancer Drugs" and other guidelines. In some embodiments, in the phase 1 clinical trial of compound (I), in order to ensure the safety of subjects, in combination with the operability of clinical trials and formulation specification, the starting dose of compound (I) for the phase I clinical trial was designed to be 5 mg. Based on the results of an animal long-term toxicity test, the maximum tolerated dose for the human body is converted into 200 mg according to the human body weight of 60 kg. In the phase 1 clinical trial of compound (I), the safety, tolerance, and efficacy of the continuous administration regimen of a daily administration dose of 5 mg-200 mg were researched. In some embodiments, for a human with a body weight of 60 kg, the daily administration dose of compound (I) is preferably 5-30 mg, more preferably 10-25 mg, yet more preferably 10-20 mg or 15-25 mg, and further preferably 10 mg, 15 mg, 20 mg, or 25 mg. The administration may be performed in a single dose once daily, in multiple doses daily, or at intervals, preferably in a single dose once daily. Preferably, the administration cycle is 21 consecutive days; optionally, the administration may be repeated for multiple cycles.

In the description and claims of the present application, the words "comprise", "include", and "contain" are intended to mean "include but not limited to", and are not intended to exclude other portions, additives, components, or steps.

In some embodiments, the compound (I) of the present application has an excellent inhibitory effect on meningioma. In a specific embodiment, the compound (I) of the present application is capable of significantly inhibiting the viability, proliferation, migration, or invasion of meningioma cells, e.g., cells of meningioma cell lines IOMM-Lee and CH157-MN. In another specific embodiment, the compound (I) of the present application has a significant promoting effect on the apoptosis of meningioma cells, e.g., cells of meningioma cell lines IOMM-Lee and CH157-MN. In some embodiments, preliminary clinical studies have shown that the compound (I) of the present application is capable of effectively treating meningioma. In some embodiments, preliminary clinical studies have shown that the compound (I) of the present application exhibits good safety and tolerance.

Thus, compared with the prior art, the present application has one or more of the following main advantages:
(1) the compound (I) has excellent inhibitory activity against meningioma, is capable of significantly inhibiting the viability, proliferation, migration, or invasion of meningioma cells, e.g., cells of meningioma cell lines IOMM-Lee and CH157-MN, and has a significant promoting effect on the apoptosis of meningioma cells; and
(2) preliminary clinical studies have shown that the compound (I) is capable of effectively treating meningioma, exhibits good safety and tolerance, and has the prospect of being developed as an anti-meningioma drug.

### BRIEF DESCRIPTION OF THE DRAWINGS

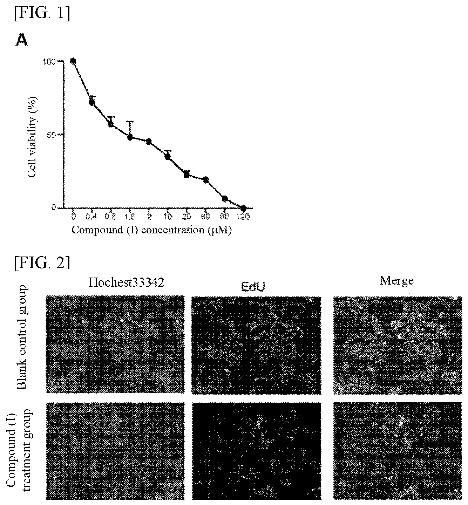

FIG. 1 shows the inhibitory effect of compound (I) on the viability of human meningioma cells.
FIG. 2 shows the inhibitory effect of compound (I) on the proliferation of human meningioma cells.

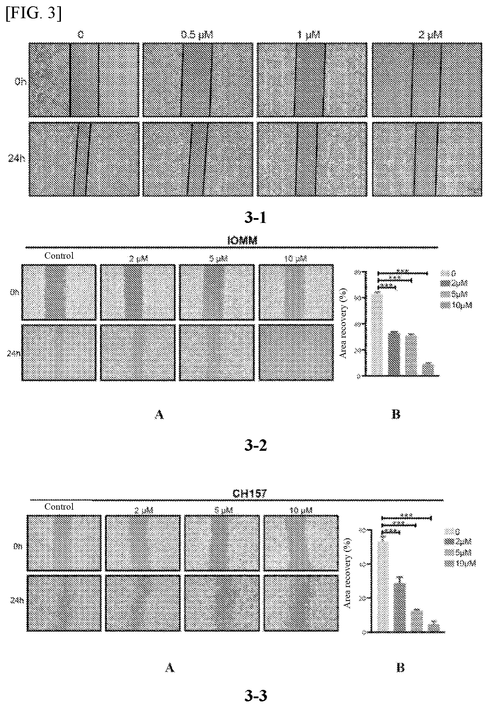

FIG. 3 shows the inhibitory effect of compound (I) on the migration of human meningioma cells. FIG. 3-1 and FIG. 3-2 show the inhibitory effect of compound (I) on the migration of human meningioma cells IOMM-Lee. FIG. 3-3 shows the inhibitory effect of compound (I) on the migration of human meningioma cells CH157-MN. In each of FIG. 3-2 and FIG. 3-3, panel A represents crystal violet staining images of human meningioma cells, and panel B shows the t-test analysis results (*** represents P < 0.001 compared with the blank control group).

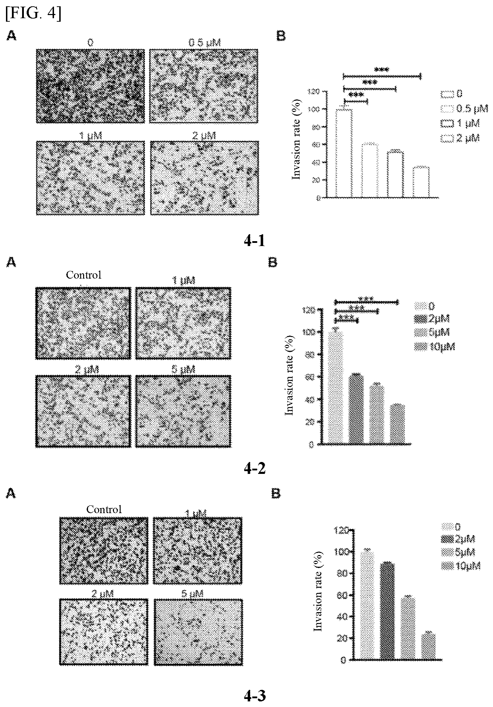

FIG. 4 shows the inhibitory effect of compound (I) on the invasion of human meningioma cells. FIG. 4-1 and FIG. 4-2 show the inhibitory effect of compound (I) on the invasion of human meningioma cells IOMM-Lee. FIG. 4-3 shows the inhibitory effect of compound (I) on the invasion of human meningioma cells CH157-MN. In each of FIG. 4-1, FIG. 4-2, and FIG. 4-3, panel A represents crystal violet staining images of human meningioma cells, and panel B shows the t-test analysis results (*** represents P < 0.001 compared with the blank control group).

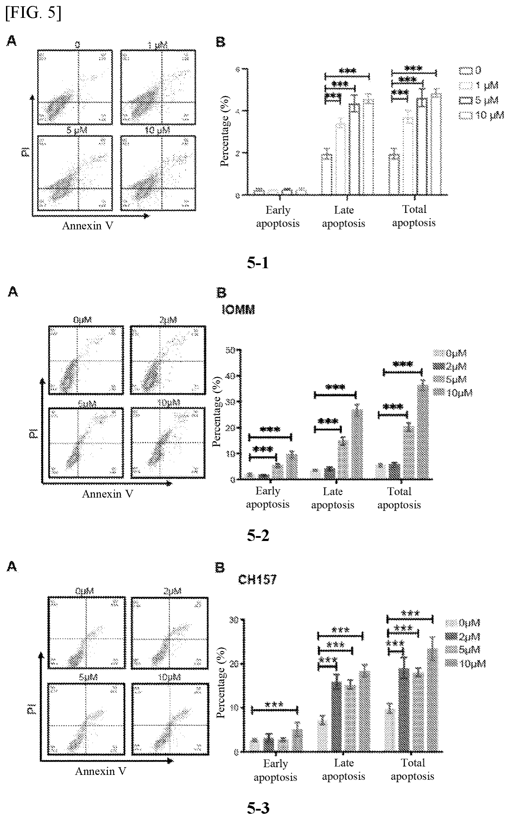

FIG. 5 shows the promoting effect of compound (I) on the apoptosis of human meningioma cells. FIG. 5-1 and FIG. 5-2 show the promoting effect of compound (I) on the apoptosis of human meningioma cells IOMM-Lee. FIG. 5-3 shows the promoting effect of compound (I) on the apoptosis of human meningioma cells CH157-MN. In each of FIG. 5-1, FIG. 5-2, and FIG. 5-3, panel A represents the flow cytometry scatter plot, and panel B shows the t-test analysis results (*** represents P < 0.001 compared with the blank control group).

### DETAILED DESCRIPTION

The present application will be further illustrated with reference to the following specific examples. It should be understood that these examples are merely intended to illustrate the present application rather than limit the scope of the present application. Experimental methods without specified conditions in the following examples are generally conducted under conventional conditions or conditions recommended by the manufacturer. Unless otherwise defined, all technical and scientific terms used herein have the same meaning as commonly understood by those of ordinary skill in the art. In addition, any methods and materials similar or equivalent to those described herein can be used in the methods of the present application. The preferred embodiments and materials described herein are for illustrative purposes only.

### Source or Preparation of Experimental Materials:

Compound (I) was prepared in-house by Shanghai Runshi Medical Technology Co., Ltd.

The positive control compound, reagents used in the experiments, and starting materials were all purchased commercially or prepared in-house.

The test substance compound (I) for *in vitro* experiments was prepared according to the method: after weighing, adding DMSO to dissolve the compound into a 10 mM solution and diluting the solution with PBS to a desired concentration at the time of use.

The test substance compound (I) formulation for the clinical experiment was prepared according to the following formula:
Specification: 20 mL: 200 mg/vial:

| Starting and auxiliary materials | Weight percentage |
|---|---|
| Compound I | 0.99 |
| Medium-chain triglyceride | 13.86 |
| Polyoxyethylene (40) hydrogenated castor oil | 56.42 |
| Polyethylene glycol 400 | 28.71 |
| Dibutyl hydroxy toluene | 0.020 |

### Preparation method:

According to the formula described above, polyoxyethylene (40) hydrogenated castor oil was melted into a liquid state in warm water at about 60 °C in advance. Medium-chain triglyceride, polyoxyethylene (40) hydrogenated castor oil, polyethylene glycol 400, and dibutyl hydroxy toluene were added into a liquid preparation tank and stirred homogeneously at 40-70 °C. The compound (I) was added, and the mixture was heated and stirred until the complete dissolution of the starting materials. The solution was aliquoted into an oral solution vial according to the specification.

### Example 1: Detection of Effect of Compound (I) on Cell Viability of Human Meningioma Cell Line by Trypan Blue Viable Cell Counting

### 1. Experimental method

Human meningioma cell line IOMM-Lee cells (purchased from ATCC) were cultured using a DMED culture medium containing 10% FBS and 100 U/mL penicillin-streptomycin double antibiotics in an incubator with 5% CO₂ at 37 °C. The cells in the logarithmic growth phase were seeded uniformly in a 6-well plate at a density of 2 × 10⁵ cells/mL and cultured for 24 h, and then the cells were grouped into a blank control group and compound (I) treatment groups at different concentrations. The compound (I) treatment concentrations were 0.4 µM, 0.8 µM, 1.6 µM, 2 µM, 10 µM, 20 µM, 60 µM, and 120 µM, and 3-4 replicate wells were set for each group. After cells in the groups were separately cultured for 24 h, the cells were digested with 0.25% trypsin, and 50 µL of each cell suspension was mixed with 50 µL of 0.4% trypan blue solution to a final trypan blue concentration of 0.2%. Subsequently, 10 µL of the uniform cell suspension was added to a counting plate and counted under a microscope for the total cell count and dead cell count. Statistical analysis was performed.

### 2. Experimental results

The results demonstrated that compared with the blank control group, after being treated for 24 h, all of the compound (I) treatment groups at different concentrations exhibited inhibitory effects on IOMM-Lee cell viability and the cell viability had a decreasing trend as the increase of compound (I) concentration. The results are as shown in Table 1 and FIG. 1. The above results demonstrate that compound (I) is capable of inhibiting the cell viability of meningioma cells IOMM-Lee.

**Table 1. Inhibitory effect of compound (I) on human meningioma cell viability**

| Concentration of compound (I) (µM) | 0 | 0.4 | 0.8 | 1.6 | 2 | 10 | 20 | 60 | 80 | 120 |
|---|---|---|---|---|---|---|---|---|---|---|
| Cell viability (%) | 99.99± 6.68 | 71.83± 4.06 | 56.75± 5.19 | 48.31± 10.48 | 45.30± 1.91 | 35.05± 3.93 | 22.55± 2.72 | 19.28± 0.52 | 6.33± 1.71 | 0±0 |

| | | | | | | | | | | |
|---|---|---|---|---|---|---|---|---|---|---|
| Note: cell viability = (total cell count - dead cell count)/total cell count × 100% | | | | | | | | | | |

### Example 2: Detection of Effect of Compound (I) on Proliferation of Human Meningioma Cell Line by EdU Assay

### 1. Experimental method

The IOMM-Lee single cell suspension in the logarithmic growth phase obtained in Example 1 was seeded in a 6-well plate at a cell density of 3 × 10⁵ cells/mL, cultured overnight, and grouped into a blank control group and a compound (I) treatment group (concentration: 1 µM; treatment duration: 24 h). Subsequently, each group was incubated with EdU (10 µM) for 2 h; the culture medium was discarded; and the cells were washed with PBS (in this experiment, PBS was used in the washing process). The cells were fixed with 4% paraformaldehyde; the stationary liquid was discarded; and the cells were washed. After that, the cells were permeabilized with 0.3% Triton X100; the permeabilization liquid was discarded; and the cells were washed. Then, a prepared click reaction solution (commercially available C0081 BeyoClick^{™} EdU Cell Proliferation Kit with Alexa Fluor 647) was added; the mixture was incubated in the dark at room temperature for 30 min; the reaction solution was discarded; and the cells were washed. The cell nuclei were stained with Hoechst 33342 fluorescent dye by adding 1× Hoechst 33342 solution at 1 mL/well, followed by incubated in the dark at room temperature for 10 min; 1× Hoechst 33342 solution was removed by pipetting; and the cells were washed 3 times. Subsequently, a fluorescence assay was performed, and the results were recorded.

### 2. Experimental results

The results are as shown in FIG. 2. In the fluorescence assay photograph, the blue fluorescence channel shows the Hochest33342 staining of the cell nuclei, and the red fluorescence channel shows the EdU staining of the cell nuclei of the proliferating cells. The merge of the two channels exhibits an overall magenta color, clearly showing the ratio of the proliferating cells to the total cells. The experimental results demonstrate that the EdU-labeled (proliferating) cells in compound (I)-treated meningioma cells IOMM-Lee were significantly fewer than those in the blank control group. The above results demonstrate that compound (I) is capable of inhibiting the cell proliferation of the meningioma cells IOMM-Lee.

### Example 3: Detection of Effect of Compound (I) on Migration of Human Meningioma Cell Line by Cell Scratch Assay

### 1. Experimental method

(1) The IOMM-Lee single cell suspension in the logarithmic growth phase was seeded in a 6-well plate at a cell density of 6 × 10⁵ cells/mL and cultured for 24 h to ensure the full covering of the entire cell culture dish by the growing cells. A cell scratch was made by a 10 µL sterile pipette tip perpendicular to the culture dish; the original culture solution was discarded; and the detached cells were pipetted by washing with PBS 3 times. The cells were cultured using a serum-free DMEM culture medium and grouped into a blank control group and compound (I) treatment groups at different concentrations, where the compound (I) treatment groups had concentrations of 0.5 µM, 1 µM, and 2 µM, respectively. The cells were observed under the bright field of a microscope and recorded at 0 h and 24 h.
(2) With reference to the experimental method (1), the concentrations in the compound (I) treatment groups were replaced by 2 µM, 5 µM, and 10 µM, respectively, and the used human meningioma cell line was IOMM-Lee or CH157-MN.

### 2. Experimental results

The experimental results demonstrate that after the IOMM-Lee cells or the CH157-MN cells were treated with compound (I) at different concentrations for 24 h, the compound (I) treatment groups exhibited significantly weaker cell scratch healing than that of the blank control group, and the cell scratch healing had a decreasing trend as the increase of compound (I) concentration. The results are as shown in FIG. 3 (FIG. 3-1, FIG. 3-2, and FIG. 3-3). The above results demonstrate that compound (I) has a significant inhibitory effect on the cell migration of meningioma cells IOMM-Lee and CH157-MN in a dose-dependent manner.

### Example 4: Detection of Effect of Compound (I) on Cell Invasion of Human Meningioma Cell Line by Transwell Chamber Assay

### 1. Experimental method

(1) The upper chamber of the pre-cooled Transwell chamber was coated with 50 µL of matrigel, and the matrigel-coated chamber was incubated in an incubator at 37 °C for 6-8 h to allow the matrigel to polymerize into a gel. After 100 µL of a serum-free culture medium was added to each well, the chamber was placed in an incubator for 1 h to allow the basement membrane hydration. The liquid in the upper chamber was removed, and the IOMM-Lee single cell suspension in the logarithmic growth phase was adjusted to a cell density of 2 × 10⁵ cells/mL using the serum-free DMEM culture medium; 200 µL of the cell suspension was added to the Transwell chamber; and the culture liquid in the lower chamber was a DMEM culture medium with 20% FBS. Compound (I) samples at different concentrations (0 µM, 0.5 µM, 1 µM, and 2 µM, respectively) were added to the chamber. The culture was finished 24 h later; and the cells in the chamber were removed with a cotton swab, fixed with 4% paraformaldehyde for 20 min, washed with PBS 3 times, stained with crystal violet for 30 min, and observed under a microscope. The invading cells were counted.
(2) With reference to the experimental method (1), the concentrations in the compound (I) treatment groups were replaced by 2 µM, 5 µM, and 10 µM, respectively, and the used human meningioma cell line was IOMM-Lee or CH157-MN.

### 2. Experimental results

The experimental results demonstrate that after the IOMM-Lee cells or the CH157-MN cells were treated with compound (I) at different concentrations for 24 h, the invasion rates significantly decreased as the increase of compound (I) concentration. The results are as shown in FIG. 4 (FIG. 4-1, FIG. 4-2, and FIG. 4-3). The above results demonstrate that compound (I) has a significant inhibitory effect on the invasion of meningioma cells IOMM-Lee and CH157-MN in a dose-dependent manner.

### Example 5: Detection of Effect of Compound (I) on Apoptosis of Human Meningioma Cell Line

### 1. Experimental method

(1) The IOMM-Lee single cell suspension in the logarithmic growth phase was seeded in a 6-well plate at a cell density of 5 × 10⁵ cells/mL, cultured overnight, and grouped into a blank control group and compound (I) treatment groups at different concentrations, with concentrations of compound (I) being 1 µM, 5 µM, and 10 µM, respectively, and 3-4 replicate wells were set. After the addition of compound (I) at different concentrations for the treatment for 24 h, (1-10) × 10⁵ cells were collected from the blank control group and each compound (I) treatment group, washed with PBS, and centrifuged. The cells were resuspended in 500 µL of 1× binding buffer. 5 µL of annexin V-FITC and 10 µL of PI were added to each tube. After gently and homogeneously mixing by vortex, the cells were incubated in the dark at room temperature for 5 min and then subjected to flow cytometry.
(2) With reference to the experimental method (1), the concentrations in the compound (I) treatment groups were replaced by 2 µM, 5 µM, and 10 µM, respectively, and the used human meningioma cell line was IOMM-Lee or CH157-MN.

### 2. Experimental results

The experimental results demonstrate that after the IOMM-Lee cells or the CH157-MN cells were treated with compound (I) at different concentrations for 24 h, the total cell apoptosis ratio increased as the increase of compound (I) concentration compared with the blank control group, and the late apoptosis significantly increased. The results are as shown in FIG. 5 (FIG. 5-1, FIG. 5-2, and FIG. 5-3). The above results demonstrate that compound (I) has a significant promoting effect on the apoptosis of meningioma cells IOMM-Lee and CH157-MN.

### Example 6: Typical Clinical Cases

Compound (I) is currently undergoing phase I clinical trials to evaluate the preliminary efficacy on patients with meningioma.

Drug: compound (I) oral solution, 10-25 mg/day, once daily.

Administration regimen for subjects: in the single-dose administration period, a single-dose administration was started after enrollment, and the subjects were observed for 3 days; in the multiple-dose administration period, the administration was performed for 21 consecutive days. If no serious adverse events occurred, subsequent treatment cycles (21 days for each cycle) of administration may begin until progressive disease (PD) or intolerable toxicity occurred.

The efficacy on tumors was evaluated according to the RANO Criteria for Meningioma (Huang, Raymond Y et al. Proposed response assessment and endpoints for meningioma clinical trials: report from the Response Assessment in Neuro-Oncology Working Group. Neuro-oncology. vol. 21, 1 (2019): 26-36. doi:10.1093/neuonc/noy137). Target lesions were selected from tumor lesions with enhancement according to the criteria and precisely measured. The tumor treatment efficacy on the target lesions was confirmed by comparing the changes in the measured values. All tumor lesions not selected as target lesions were labeled as non-target lesions, which were observed at each tumor assessment, and the changes were described and compared. In addition, the improvement and deterioration of clinical symptoms of patients and the change in steroid hormone doses for symptomatic treatment of neurological symptoms were also used as evaluation indexes in tumor assessment. A tumor assessment was performed at baseline prior to the first administration and then performed once every 6 weeks during the administration until progressive disease (PD) occurred or a new anti-tumor treatment started.

In total, 34 meningioma patients with postoperative residual tumor, recurrence, or progressive disease received the treatment of compound (I), and most of the patients exhibited tumor shrinkage and stable or improved clinical symptoms after administration. By Jan. 16, 2023, 5 patients completed the tumor assessment, among which were 2 PR cases, 1 MR case, and 2 SD cases. By Feb. 2, 2024, 32 patients completed the tumor assessment, among which were 4 PR cases, 6 MR cases, and 18 SD cases. As assessed according to the RANO-criteria for meningioma, the objective remission rate was 29.41%, the disease control rate was 82.4%, and 26 patients exhibited tumor shrinkage after administration, accounting for 76.5% of all patients. The adverse events associated with the administration of compound (I) were mostly mild and moderate, and adverse events with an incidence of greater than 20% were: hypertension, decreased platelet count, increased blood lactate dehydrogenase, sinus bradycardia, proteinuria, increased aspartate aminotransferase, increased alanine aminotransferase, hypokalemia, hyperlipidemia, decreased neutrophil count, decreased leukocyte count, diarrhea, and anemia.

### Typical case 1:

A patient, female, 59 years old, was diagnosed with anaplastic meningioma having a pathological type of anaplastic (malignant) meningioma (CNS WHO grade III) with brain tissue infiltration.

On Jul. 12, 2015, the patient received right middle intracranial meningioma resection and then received no chemoradiotherapy as well as targeted therapy, and the patient underwent regular follow-up. In November-December 2020, enhanced nuclear magnetic resonance of the head showed tumor progression. On Jan. 18, 2021, the patient received meningioma resection, and the tumor base was found to be attached to the middle cranial fossa and the tumor size was 4 × 4 × 4.5 cm. On Jul. 4, 2022, the patient took part in the clinical trial of injectable honokiol liposome (HK), and the last treatment time was Jul. 31, 2022. On Aug. 9, 2022, the patient had deteriorated clinical symptoms, and was withdrawn from the trial with comprehensive assessment. On Aug. 16, 2022, the patient took part in the phase I clinical trial of compound (I). On Aug. 31, 2022, the patient started to take compound (I) oral solution at a dose of 15 mg once daily.

**Efficacy evaluation of tumor treatment:** A single-dose administration started after enrollment and no adverse events were observed for 3 days. Then, a multiple-dose administration started until the completion of two-cycle administration. The subject reported significantly mitigated head-distending pain. Head MRI showed significant tumor shrinkage (C2 target lesion shrinkage by 56%) and that non-target lesions had neither recovery nor progression. The patient had no hormone administration; the clinical status was stable or improved; and the overall efficacy was assessed as PR. No adverse events were reported during the treatment.

### Typical case 2:

A patient, female, 46 years old, was diagnosed with meningioma (fibrous) having a pathological type of meningioma (fibrous), WHO grade I.

On May 4, 2017, the patient received surgery. In June 2020, the patient received surgery again, and in August 2020 and March 2021, the patient received gamma knife treatment. On Mar. 22, 2022, the patient received camrelizumab intravenous treatment, but developed intolerance, visual deterioration, and gradual blindness.

On Nov. 7, 2022, the patient took part in the phase I clinical trial of compound (I). On Nov. 16, 2022, the patient started to take compound (I) oral solution at a dose of 15 mg once daily. Baseline physical examination: blindness in both eyes, light sensation only.

**Efficacy evaluation of tumor treatment:** A single-dose administration started after enrollment and no adverse events were observed for 3 days. Then, a multiple-dose administration started. After two cycles, the subject came for a visit, and exhibited vision recovery and self-care recovery. Head MRI showed significant tumor shrinkage (C2 target lesion shrinkage by 36%) and no non-target lesions. The patient had no hormone administration; the clinical status was stable or improved; and the overall efficacy was assessed as MR. The main adverse event during the treatment was mild or moderate hypertension, which was recovered after administration was suspended.

### Typical case 3:

A patient, female, 50 years old, was diagnosed meningioma.

In May 2018, the patient received right parieto-occipital tumor resection; postoperative pathology showed anaplastic meningioma, grade III; and a postoperative synchronized chemoradiotherapy was performed. In March 2021, the patient received the 2^{nd} surgery due to tumor recurrence; postoperative pathology showed atypical meningioma, grade II; no postoperative chemoradiotherapy was performed thereafter. In September 2022, the patient received the 3^{rd} right parieto-occipital craniotomy; postoperative pathology showed to be in accordance with a malignant meningioma diagnosis.

On Oct. 17, 2022, the patient took part in the phase I clinical trial of compound (I). On Oct. 26, 2022, the patient started to take compound (I) oral solution at a dose of 15 mg via oral administration once daily.

**Efficacy evaluation of tumor treatment:** Head MRI showed significant tumor shrinkage (C2 target lesion shrinkage by 56%) and no non-target lesions. The patient had no hormone administration; the clinical status was stable or improved; and the overall efficacy was assessed as PR. The main adverse event during the treatment was mild or moderate hypertension, which was recovered after taking a hypotensor.

### Typical case 4:

A patient, female, 55 years old, was diagnosed with meningioma.

In May 2021, the patient was diagnosed with meningioma and received right frontal cranial meningioma resection. In June 2022, the patient received the 2^{nd} surgery due to recurrence; postoperative pathology showed atypical meningioma, WHO grade II; the patient received postoperative gamma knife radiotherapy and then received no treatment thereafter.

On May 5, 2023, the patient took part in the phase I clinical trial of compound (I), and on May 11, 2023, the patient started to take compound (I) oral solution at a dose of 20 mg via oral administration once daily.

**Efficacy evaluation of tumor treatment:** In the patient screening period, the patient had an 18 × 12 mm irregular annular enhancement near the right parietal lobe superior sagittal sinus. After 6 cycles of administration, head MRI showed tumor shrinkage (target lesion shrinkage by 61.47%) and a stable non-target lesion on the right parietal lobe. The patient had no hormone administration; the clinical status was stable or improved; the overall efficacy was assessed as PR; and the target lesion continued to shrink during the subsequent efficacy assessment. Currently, the patient continued to be treated after 9 months of administration, and the main adverse events during the treatment were mild proteinuria, decreased platelet count, decreased neutrophil count, decreased leucocyte count, and increased alanine aminotransferase, which were recovered after symptomatic treatment.

### Typical case 5:

A patient, male, 45 years old was diagnosed with meningioma.

In 2017, abnormality on the left frontotemporal region was found in the patient, who then received the first left frontotemporal space-occupying lesion resection on Sep. 14, 2017; postoperative pathology showed meningothelial meningioma, WHO grade I. In July 2019, the tumor recurred, and the patient received left temporal region meningioma resection; postoperative pathology showed atypical meningioma, WHO grade II. In April 2020, the tumor recurred again, and the patient received the third cranio-cervico-facial combined radical surgery; postoperative pathology showed atypical meningioma, WHO grade II; then a postoperative synchronized chemoradiotherapy was performed. On Dec. 29, 2022, the patient received the fourth brain lesion resection; postoperative pathology showed papillary meningioma, WHO grade III. In August 2023, the patient received X-ray stereotactic radiotherapy for 20 GUy/4 fx.

On Oct. 19, 2023, the patient took part in the phase I clinical trial of compound (I), and on Oct. 25, 2023, the patient started to take compound (I) oral solution at a dose of 25 mg via oral administration once daily.

**Efficacy evaluation of tumor treatment:** In the screening period, the patient had a 60 × 32 × 27 mm bifrontal parafalcine enhancing lesion. After 6 cycles of administration, head MRI showed tumor shrinkage (target lesion shrinkage by 36.73%) compared with the prior size and stable non-target lesions on the left temporal lobe and left orbital. The patient had no hormone administration; the clinical status was stable or improved; the overall efficacy was assessed as MR; and the target lesion continued to shrink during the subsequent efficacy assessment. The main adverse events during the treatment were mild hypertension, decreased platelet count, decreased neutrophil count, and increased alanine aminotransferase, which were mitigated/recovered after symptomatic treatment.

The above preliminary clinical treatment data demonstrate that the compound (I) is capable of effectively treating meningioma and exhibits good safety and tolerance.

**The full names of the abbreviations used in the present application are as follows:**

| abbreviation | Full name |
|---|---|
| DMEM | dulbecco's modified eagle medium |
| µM | µmol/L |
| FBS | Fetal Bovine Serum |
| EdU | 5-Ethynyl-2'- deoxyuridine |
| PBS | phosphate buffered saline |
| PI | propidium iodide |
| CNS | central nervous system |
| ORR | objective response rate |
| CR | complete response |
| PR | Partial Response |
| MR | Minor Response |
| SD | stable disease |
| PD | progressive disease |
| MRI | Magnetic Resonance Imaging |
| FLAIR | fluid attenuated inversion recovery |

It should be understood that although the present application describes the involved invention in the specific forms described above, the present disclosure is not limited to the specific content described in these forms. It will be apparent to those skilled in the art that, without departing from the spirit of the present disclosure described herein, various equivalent changes may be made to the technical features involved in the present disclosure, and these changes shall fall within the scope of the present disclosure.

## Claims

1. Use of a compound (I) or a pharmaceutically acceptable salt thereof in the manufacture of a medicament for treating meningioma, wherein the compound (I) has the following structure:

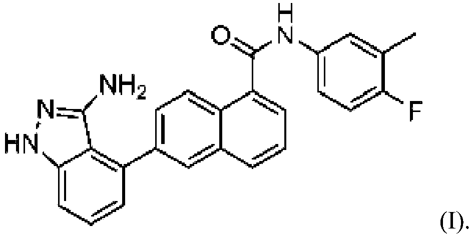

2. The use according to claim 1, wherein the meningioma comprises WHO grade I meningioma, WHO grade II meningioma, and WHO grade III meningioma categorized according to clinical and pathological characteristics.

3. The use according to claim 2, wherein the WHO grade I meningioma is a benign meningioma and is preferably selected from one or more of meningothelial meningioma, fibrous meningioma, transitional meningioma, psammomatous meningioma, angiomatous meningioma, microcystic meningioma, secretory meningioma, lymphoplasmacyte-rich meningioma, and metaplastic meningioma; and/or
the WHO grade II meningioma is an untypical meningioma and is preferably selected from one or more of atypical meningioma, clear cell meningioma, and chordoid meningioma; and/or
the WHO grade III meningioma is a malignant meningioma and is preferably selected from one or more of rhabdoid meningioma, papillary meningioma, and anaplastic meningioma.

4. The use according to claim 1, wherein the meningioma is a meningioma that failed treatment with other therapeutic means or recurred, wherein the other therapeutic means comprise surgery, radiotherapy, and treatment with an anti-tumor drug.

5. The use according to claim 4, wherein the anti-tumor drug comprises a chemotherapeutic drug, a targeted therapeutic drug, and an immunotherapeutic drug.

6. The use according to claim 5, wherein the anti-tumor drug is selected from one or more of camrelizumab, bevacizumab, sunitinib, everolimus, etoposide, temozolomide, sirolimus, and honokiol.

7. A pharmaceutical composition for treating meningioma, comprising a therapeutically effective amount of a compound (I) or a pharmaceutically acceptable salt thereof and a pharmaceutically acceptable carrier, wherein the compound (I) has the following structure:

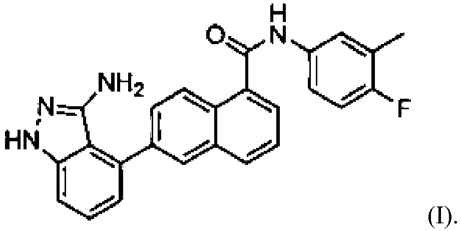

8. The pharmaceutical composition according to claim 7, wherein the pharmaceutical composition is an oral formulation, preferably an oral solution.

9. A method for treating meningioma in an individual, comprising:
administering to the individual having meningioma a therapeutically effective amount of a compound (I) or a pharmaceutically acceptable salt thereof, or the pharmaceutical composition according to claim 7 or 8;
wherein the compound (I) has the following structure:

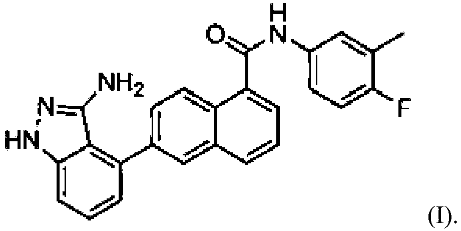

10. The method according to claim 9, wherein the therapeutically effective amount is: a daily administration dose of the compound (I) of 5 mg-30 mg, preferably 10 mg-25 mg, more preferably 10 mg-20 mg or 15 mg-25 mg, and further preferably 10 mg, 15 mg, 20 mg, or 25 mg.

11. The method according to claim 10, wherein the compound (I) is administered in a single dose once daily, in multiple doses daily, or at intervals, preferably in a single dose once daily.

12. The method according to claim 10, wherein an administration cycle of the compound (I) is 21 consecutive days; optionally, the administration can be repeated for multiple cycles.

13. The method according to claim 9, wherein the meningioma comprises WHO grade I meningioma, WHO grade II meningioma, and WHO grade III meningioma categorized according to clinical and pathological characteristics; optionally,
the WHO grade I meningioma is a benign meningioma and is preferably selected from one or more of meningothelial meningioma, fibrous meningioma, transitional meningioma, psammomatous meningioma, angiomatous meningioma, microcystic meningioma, secretory meningioma, lymphoplasmacyte-rich meningioma, and metaplastic meningioma; and/or
the WHO grade II meningioma is an untypical meningioma and is preferably selected from one or more of atypical meningioma, clear cell meningioma, and chordoid meningioma; and/or
the WHO grade III meningioma is a malignant meningioma and is preferably selected from one or more of rhabdoid meningioma, papillary meningioma, and anaplastic meningioma.

14. A compound (I) or a pharmaceutically acceptable salt thereof, or a pharmaceutical composition comprising the compound (I) or the pharmaceutically acceptable salt thereof for use in the treatment of meningioma, wherein the compound (I) has the following structure:

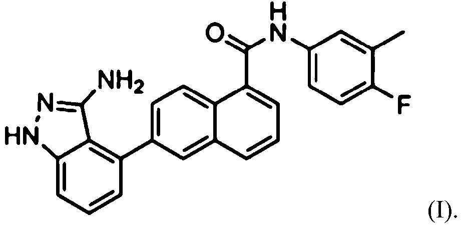

15. The compound (I) or the pharmaceutically acceptable salt thereof, or the pharmaceutical composition comprising the compound (I) or the pharmaceutically acceptable salt thereof for use according to claim 14, wherein the meningioma comprises WHO grade I meningioma, WHO grade II meningioma, and WHO grade III meningioma categorized according to clinical and pathological characteristics; optionally,
the WHO grade I meningioma is a benign meningioma and is preferably selected from one or more of meningothelial meningioma, fibrous meningioma, transitional meningioma, psammomatous meningioma, angiomatous meningioma, microcystic meningioma, secretory meningioma, lymphoplasmacyte-rich meningioma, and metaplastic meningioma; and/or
the WHO grade II meningioma is an untypical meningioma and is preferably selected from one or more of atypical meningioma, clear cell meningioma, and chordoid meningioma; and/or
the WHO grade III meningioma is a malignant meningioma and is preferably selected from one or more of rhabdoid meningioma, papillary meningioma, and anaplastic meningioma.
